# EUROPEAN PATENT SPECIFICATION

(11) **EP 4 289 471 B1**
(45) Date of publication and mention of the grant of the patent: **30.07.2025**
(21) Application number: 21924877.0
(22) Date of filing: 24.12.2021
(51) Int. Cl.: A61N 5/06

(54) **LIGHT IRRADIATION HAIR REMOVAL DEVICE**
LICHTBESTRAHLUNGSVORRICHTUNG ZUR HAARENTFERNUNG
DISPOSITIF D'ÉPILATION PAR IRRADIATION LUMINEUSE

(30) Priority: 05.02.2021 JP 2021017229
(43) Date of publication of application: 13.12.2023
(73) Proprietor: Panasonic Intellectual Property Management Co., Ltd., Kadoma-shi, Osaka 571-0057 (JP)
(72) Inventor: SAKAMOTO, Ryohei, Kadoma-shi, Osaka 571-0057 (JP); KASUGAI, Hideki, Kadoma-shi, Osaka 571-0057 (JP)
(74) Representative: Grünecker Patent- und Rechtsanwälte PartG mbB
(86) International application number: PCT/JP2021/048149
(87) International publication number: WO 2022/168492

(56) References cited:
- WO-A2-2005/074830
- CN-A- 112 155 725
- JP-A- 2003 126 276
- JP-A- 2006 149 489
- JP-A- 2008 289 812
- JP-A- 2012 239 874
- JP-A- 2013 111 391
- JP-A- 2013 111 391
- US-A- 6 165 171
- US-A1- 2013 184 693
- US-B1- 6 168 589

## Description

### TECHNICAL FIELD

The present disclosure relates to a light irradiation hair removal device.

### BACKGROUND ART

Conventionally, a light irradiation hair removal device that emits light to remove hair is known. The light irradiation hair removal device promotes a discharge of hair by irradiating a skin surface of a user with a light having a specific wavelength and causing the light to act on melanin of hair follicles. As a light irradiation hair removal device, for example, a device as shown in PTL 1 is known.

PTL 1 discloses a light irradiation hair removal device having a light source that causes a processing light and a sensing light to be incident on a target object, a light detector that detects the sensing light for sensing the target object, and a control unit for controlling the light source. The control unit determines absorption of the sensing light from the detected sensing light, and controls the light source such that the processing light is generated depending on the determined absorption. The processing light has a wavelength in a range from 570 nm to 1200 nm, an energy density in a range from 2 J/cm² to 30 J/cm², and a pulse duration within 1 ms to 600 ms.

### Citation List

### Patent Literature

PTL 1: Japanese Patent No. 5715128

A further relevant prior art is formed by document US 2013/0184693 A1.

### SUMMARY OF THE INVENTION

In a conventional light irradiation hair removal device, an application of light is controlled depending on characteristics of the target object to be irradiated with light. However, a sensitivity to pain of the user varies, and the user tends to feel pain as the skin is irradiated with stronger light to obtain a sufficient hair removal effect. Therefore, even when the application of light is controlled depending on the characteristics of the target object, the user may feel uncomfortable in a case where the skin is irradiated with stronger light.

The present disclosure provides a light irradiation hair removal device capable of reducing discomfort associated with light irradiation.

The invention is defined in the appended set of claims. A light irradiation hair removal device according to one aspect of the present disclosure includes a light source, a skin cooling unit, and a push switch. The light source intermittently emits light including a first irradiation light and a second irradiation light emitted for a longer time than the first irradiation light before the first irradiation light, and having a wavelength from 400 nm to 1200 nm inclusive. The skin cooling unit faces the light source, transmits light emitted from the light source, and cools a skin in a case where it comes into contact with the skin. The push switch includes a pressing unit surrounding a periphery of the light source and the skin cooling unit. In a case where the pressing unit is not pressed, it protrudes toward a direction opposite to the light source against the skin cooling unit from a surface of the skin cooling unit in contact with the skin. In a case where the pressing unit is pressed, a surface pressed by the skin moves toward the direction of the light source against the skin cooling unit. The push switch switches between emission and non-emission of light from the light source such that light is emitted from the light source during at least a part of time while the pressing unit is pressed, and light is not emitted from the light source while the pressing unit is not pressed. The first irradiation light has a maximum irradiance that is the largest irradiance of light emitted by the light source in an intermittent manner, and the second irradiation light has an irradiance smaller than the maximum irradiance.

According to the present disclosure, it is possible to obtain a light irradiation hair removal device capable of reducing discomfort associated with light irradiation.

### BRIEF DESCRIPTION OF THE DRAWINGS

Fig. 1 is a cross-sectional view illustrating a configuration of a light irradiation hair removal device according to the present exemplary embodiment.
Fig. 2 is a cross-sectional view taken along line II-II in Fig. 1.
Fig. 3 is a perspective view illustrating an example of a schematic arrangement state of a light source according to the present exemplary embodiment.
Fig. 4 is a graph illustrating an example of a relationship between irradiation time and irradiance of light emitted from the light source.
Fig. 5 is a control block diagram according to a controller.
Fig. 6 is a cross-sectional view illustrating an example of a state before use of the light irradiation hair removal device.
Fig. 7 is a cross-sectional view illustrating an example of a state of the light irradiation hair removal device before a push switch is pressed.
Fig. 8 is a cross-sectional view illustrating an example of a state of the light irradiation hair removal device after the push switch is pressed.
Fig. 9 is a cross-sectional view illustrating an example of a state of the light irradiation hair removal device while a skin is irradiated with light.
Fig. 10 is a graph illustrating another example of the relationship between the irradiation time and the irradiance of light emitted from the light source.

### DESCRIPTION OF EMBODIMENT

Hereinafter, exemplary embodiments will be described in detail with reference to the drawings. However, detailed descriptions more than necessary may be omitted. For example, detailed descriptions of already well-known matters or redundant descriptions of substantially the same configuration may be omitted.

Note that, the accompanying drawings and the following description are only presented to help those skilled in the art fully understand the present disclosure, and are not intended to limit the subject matters described in the scope of claims.

Further, in the following exemplary embodiment, up-down direction Z of light irradiation hair removal device 1 is defined with an emission port as an upward direction and a direction opposite to the emission port as a downward direction. Further, a direction in a horizontal direction of light irradiation hair removal device 1 is defined as width direction Y, and a direction orthogonal to up-down direction Z and width direction Y is defined as front-back direction X.

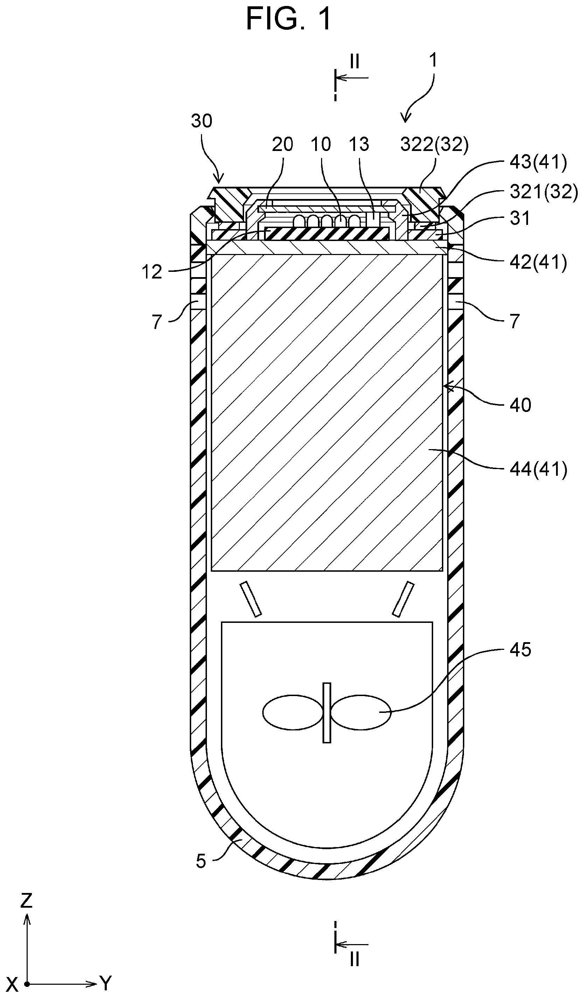

Hereinafter, light irradiation hair removal device 1 according to the present exemplary embodiment will be described with reference to Figs. 1 to 9.

### [Configuration]

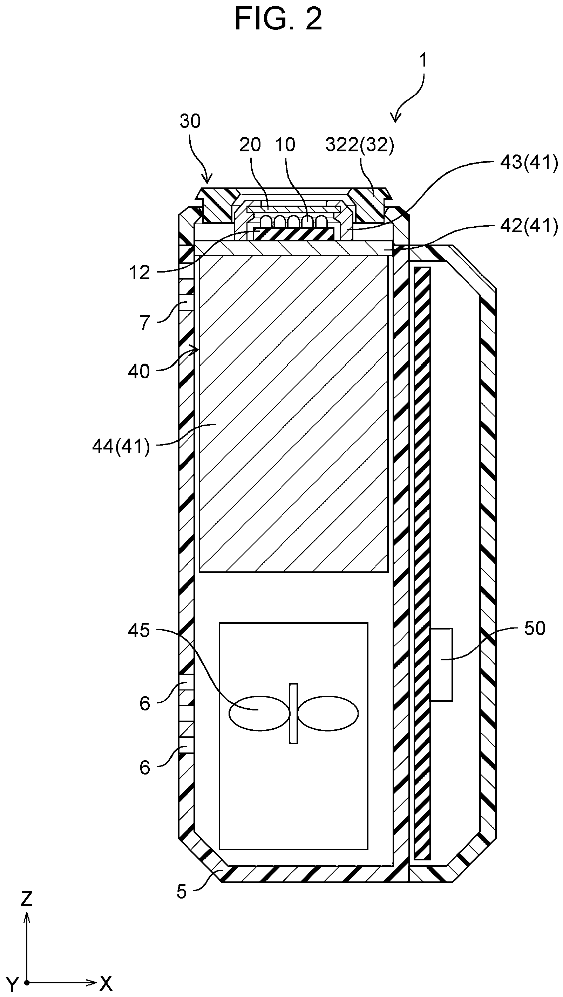

Fig. 1 is a cross-sectional view illustrating the configuration of light irradiation hair removal device 1 according to the present exemplary embodiment, and Fig. 2 is a cross-sectional view taken along line II-II in Fig. 1. As shown in Figs. 1 and 2, light irradiation hair removal device 1 includes housing 5, light source 10, temperature sensor 13, skin cooling unit 20, push switch 30, cooler 40, and controller 50.

One end of housing 5 is provided with an opening portion serving as a light emission port of light irradiation hair removal device 1. Light source 10 is provided in the opening portion of housing 5, and emits light on skin S of a person. Further, a bottom portion is formed on a side of housing 5 opposite to light source 10. Housing 5 is provided with a plurality of first opening portions 6 and a plurality of second opening portions 7, and external air is taken in from the plurality of first opening portions 6 and discharged from the plurality of second opening portions 7. Inside housing 5, light source 10, temperature sensor 13, skin cooling unit 20, push switch 30, cooler 40, and controller 50 are accommodated.

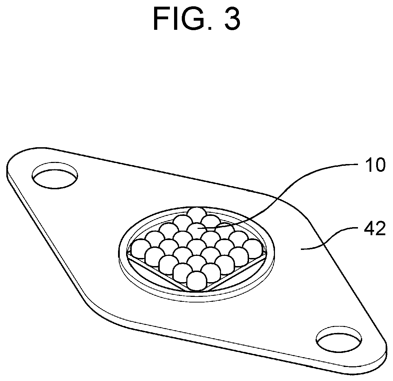

Fig. 3 is a perspective view illustrating an example of a schematic arrangement state of light source 10 according to the present exemplary embodiment. Note that, in Fig. 3, configurations of temperature sensor 13, skin cooling unit 20, push switch 30, and cooler 40 are partially omitted. As shown in Figs. 1 to 3, in the present exemplary embodiment, light source 10 includes a plurality of light emitting diodes (LEDs). The LEDs are mounted on substrate 12 in a state of being spaced at substantially equal intervals. Light source 10 is electrically connected to a power supply (not illustrated), and when power is supplied from the power supply, light is emitted from light source 10.

Light source 10 intermittently emits light including first irradiation light L1 and second irradiation light L2 emitted before first irradiation light L1. First irradiation light L1 has a maximum irradiance that is the largest irradiance of light emitted by light source 10 in an intermittent manner. Second irradiation light L2 has an irradiance smaller than the maximum irradiance. Second irradiation light L2 is emitted for a longer time than first irradiation light L1.

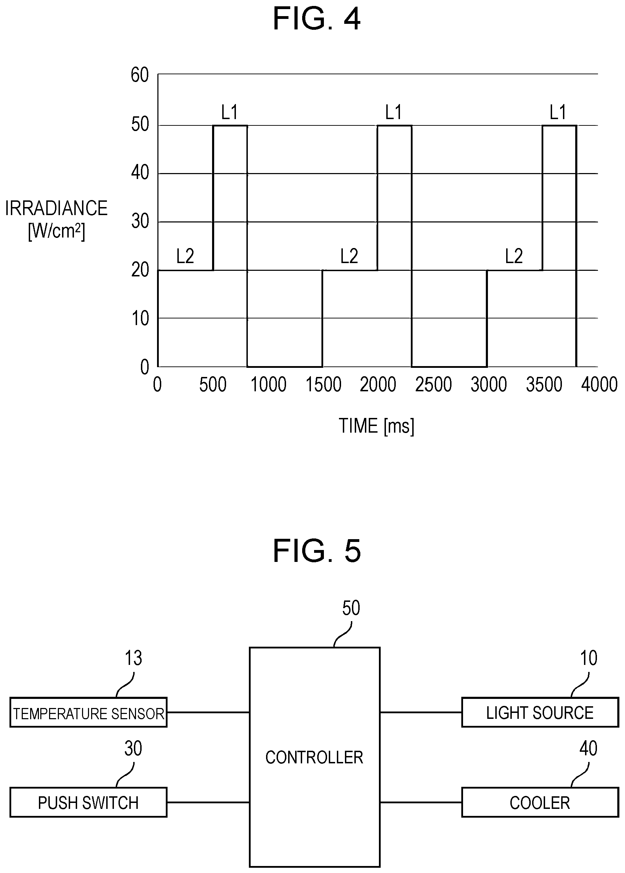

Fig. 4 is a graph illustrating an example of a relationship between the irradiation time and irradiance of light emitted from light source 10. As shown in Fig. 4, first, second irradiation light L2 is emitted from light source 10. The irradiance of second irradiation light L2 is 20 W/cm², and the irradiation time of second irradiation light L2 is 500 ms. Next, first irradiation light L1 is emitted from light source 10 following the emission of second irradiation light L2. The irradiance of first irradiation light L1 is 50 W/cm², and the irradiation time of first irradiation light L1 is 300 ms. Therefore, total irradiation time of second irradiation light L2 and first irradiation light L1 is 800 ms. After second irradiation light L2 and first irradiation light L1 are emitted, light emission by light source 10 is stopped. Off time of light source 10 is 700 ms. Next, second irradiation light L2 and first irradiation light L1 are emitted again from light source 10 in this order. Light source 10 intermittently emits light including second irradiation light L2 and first irradiation light L1 by repeatedly turning on and off light including second irradiation light L2 and first irradiation light L1.

First irradiation light L1 may have a constant irradiance in a predetermined time period as shown in Fig. 4, but the irradiance may increase or decrease with a lapse of time. The irradiance of first irradiation light L1 may be within a range from more than 90% to less than or equal to 100% relative to the maximum irradiance.

The irradiance of light emitted from light source 10 is preferably more than 0 W/cm² and less than or equal to 50 W/cm². By setting the irradiance within the above range, it is possible to suppress a rise in skin temperature due to light irradiation, and to reduce skin irritation. The irradiance may be less than or equal to 45 W/cm², or less than or equal to 40 W/cm². Further, the irradiance of light emitted from light source 10 is preferably more than or equal to 15 W/cm². By irradiating the hair with light at an irradiance more than or equal to 15 W/cm², it is possible to achieve a good hair removal effect on hair from an early growth period to a growth period. An irradiance may be more than or equal to 20 W/cm², more than or equal to 25 W/cm², or more than or equal to 30 W/cm².

The irradiance of first irradiation light L1 is preferably more than 0 W/cm² and less than or equal to 50 W/cm². By setting the irradiance within the above range, it is possible to suppress a rise in skin temperature due to light irradiation, and to reduce skin irritation. Further, the irradiance of first irradiation light L1 is preferably more than or equal to 15 W/cm². By irradiating the hair with light at an irradiance more than or equal to 15 W/cm², it is possible to achieve a good hair removal effect on hair from an early growth period to a growth period. The irradiance may be more than or equal to 30 W/cm², or more than or equal to 40 W/cm².

Second irradiation light L2 has an irradiance smaller than the maximum irradiance. As a result, since a user gets used to the pain due to the irradiation of second irradiation light L2, it is difficult for the user to feel pain even when irradiated with first irradiation light L1, and the user's discomfort can be reduced. The irradiance of second irradiation light L2 may be more than or equal to 10 W/cm². Further, the irradiance of second irradiation light L2 may be less than or equal to 40 W/cm², or less than or equal to 30 W/cm².

The irradiance of second irradiation light L2 may be less than or equal to 90% relative to the maximum irradiance. As a result, since the user gets used to the pain by the irradiation of second irradiation light L2, it is difficult for the user to feel pain even when irradiated with first irradiation light L1, and the user's discomfort can be further reduced. The irradiance of second irradiation light L2 may be less than or equal to 80%, less than or equal to 70%, less than or equal to 60%, or less than or equal to 50% relative to the maximum irradiance. Further, the irradiance of second irradiation light L2 may be more than or equal to 10%, more than or equal to 20%, or more than or equal to 30% relative to the maximum irradiance.

Respective irradiation time of light emitted from light source 10 in an intermittent manner, which is the total irradiation time of light in one cycle, is preferably from 500 ms to 1000 ms inclusive. By irradiating the hair with light for more than or equal to 500 ms, it is possible to achieve a good hair removal effect on hair from the early growth period to the growth period. Further, by irradiating the skin with light for less than or equal to 1000 ms, it is possible to suppress a rise in skin temperature due to light irradiation and to reduce skin irritation. The irradiation time may be more than or equal to 600 ms. Further, the irradiation time may be less than or equal to 900 ms, or less than or equal to 800 ms.

Second irradiation light L2 is emitted for a longer time than first irradiation light L1. As a result, hair removal can be promoted by increasing the temperature of hair follicles while reducing the irritation to skin S due to light irradiation. The irradiation time of first irradiation light L1 is preferably more than 0 ms and less than 500 ms. The irradiation time of first irradiation light L1 may be more than or equal to 100 ms, or more than or equal to 200 ms. Further, the irradiation time of first irradiation light L1 may be less than or equal to 400 ms. The irradiation time of second irradiation light L2 is preferably more than 0 ms and less than or equal to 500 ms. The irradiation time of second irradiation light L2 may be more than or equal to 100 ms, more than or equal to 200 ms, more than or equal to 300 ms, or more than or equal to 400 ms.

A ratio of the irradiation time of first irradiation light L1 to the irradiation time of each light emitted from light source 10 in an intermittent manner (which is the total irradiation time of each light) is preferably from 0.1 to 0.5 inclusive. By setting the ratio within the above range, it is possible to further reduce irritation to skin S due to light irradiation. The ratio may be more than or equal to 0.2, or more than or equal to 0.3. Further, the ratio may be less than or equal to 0.45, or less than or equal to 0.4.

A ratio of the irradiation time of second irradiation light L2 to the irradiation time of each light emitted from light source 10 in an intermittent manner (which is the total irradiation time of each light) is preferably from 0.5 to 0.8 inclusive. By setting the ratio within the above range, it is possible to further promote hair removal by increasing the temperature of hair follicles while further reducing irritation to skin S due to light irradiation. The ratio may be more than or equal to 0.55. Further, the ratio may be less than or equal to 0.7.

A ratio of the total irradiation time of first irradiation light L1 and second irradiation light L2 to the irradiation time of each light emitted from light source 10 in an intermittent manner (which is the total irradiation time of each light) may be more than or equal to 0.6, more than or equal to 0.7, more than or equal to 0.8, or more than or equal to 0.9.

A ratio of the irradiation time of first irradiation light L1 to the irradiation time of second irradiation light L2 is preferably more than 0.5 and less than or equal to 0.9. By setting the ratio within the above range, it is possible to further reduce irritation to skin S due to light irradiation. The ratio may be more than or equal to 0.55. Further, the ratio may be less than or equal to 0.8, or less than or equal to 0.7.

Light source 10 emits light having a wavelength from 400 nm to 1200 nm inclusive. When the light as described above is emitted on skin S, melanin of hair follicles absorbs the light and generates heat. Then, hair matrixes included in the hair follicles are damaged by the heat, and a hair discharge is promoted. A wavelength of light may be more than or equal to 500 nm, more than or equal to 600 nm, more than or equal to 700 nm, or more than or equal to 800 nm. Further, a wavelength of light may be less than or equal to 1000 nm, or less than or equal to 900 nm. The light emitted from light source 10 may be light having a peak wavelength within a range from 400 nm to 1200 nm inclusive. Even in a case where light has a peak wavelength within the range as described above, the emitted light may contain a wavelength component outside the above range. Further, each of the LEDs does not need to have the same wavelength spectrum, and LEDs that emit light of different wavelength spectra may be used in combination. Note that, the wavelength is a wavelength of light emitted in a case where the temperature of light source 10 is 25°C.

Energy of each pulsed light emitted from light source 10 is preferably from 9 J/cm² to 50 J/cm² inclusive. When the energy is more than or equal to 9 J/cm², a good hair removal effect can be achieved. Further, in light irradiation hair removal device 1 including skin cooling unit 20, when the energy is less than or equal to 50 J/cm², a rise in skin temperature due to light irradiation can be suppressed. Therefore, skin S is cooled more reliably, and skin irritation can be reduced.

Skin cooling unit 20 is disposed at a position facing light source 10. Skin cooling unit 20 may be in contact with light source 10, or may be disposed with a space from light source 10. Further, skin cooling unit 20 is provided so as to be in contact with skin S on a side opposite to light source 10. Skin cooling unit 20 is made of a material having translucency. When light source 10 emits light, skin cooling unit 20 transmits light emitted from light source 10, and skin S is irradiated with light transmitted through skin cooling unit 20. Skin cooling unit 20 is, for example, a plate having translucency, and in the present exemplary embodiment, skin cooling unit 20 of a disk is used.

Skin cooling unit 20 is preferably made of a material that is difficult to absorb light emitted from light source 10. Specifically, a total light transmittance of skin cooling unit 20 is preferably more than or equal to 80%. When the total light transmittance is more than or equal to 80%, most of light emitted from light source 10 can be transmitted through skin cooling unit 20. Therefore, a large amount of light can reach melanin, which can promote the hair removal effect. Further, since the amount of light absorbed by skin cooling unit 20 and converted into heat can be reduced, a temperature rise of skin cooling unit 20 can be suppressed. From a viewpoint of making it difficult for skin cooling unit 20 to absorb light, the total light transmittance is more preferably more than or equal to 90%, still more preferably more than or equal to 95%, and particularly preferably more than or equal to 99%. An upper limit value of the total light transmittance is 100%. The total light transmittance can be measured according to JIS K7361-1:1997.

A refractive index of skin cooling unit 20 is preferably more than or equal to 1.7. When the refractive index of skin cooling unit 20 is more than or equal to 1.7, light from light source 10 is not easily absorbed by skin cooling unit 20. Skin cooling unit 20 becomes easier to transmit light as the refractive index value increases. Therefore, the refractive index is more preferably more than or equal to 1.8, still more preferably more than or equal to 1.9, and particularly preferably more than or equal to 2.0. The upper limit value of the refractive index is not particularly limited, but may be 10. The refractive index can be measured by minimum deviation method according to JIS B7071-1:2015.

Skin cooling unit 20 cools skin S when it comes into contact with skin S. Skin cooling unit 20 preferably includes a material having a high thermal conductivity. A thermal conductivity of skin cooling unit 20 is preferably more than or equal to 1 W/mK. When the thermal conductivity is more than or equal to 1 W/mK, even when skin cooling unit 20 is heated by light from light source 10 and skin S, heat is easily dissipated, and thus skin S can be effectively cooled. As a value of the thermal conductivity increases, the thermal conductivity of skin cooling unit 20 tends to increase, and a cooling effect of skin cooling unit 20 tends to be better. Therefore, from a viewpoint of cooling efficiency, the thermal conductivity of skin cooling unit 20 is more preferably more than or equal to 2 W/mK, still more preferably more than or equal to 10 W/mK, particularly preferably more than or equal to 30 W/mK, and most preferably more than or equal to 100 W/mK. The upper limit value of the thermal conductivity is not particularly limited, but may be 100,000 W/mK. The thermal conductivity can be measured by laser flash method according to JIS R1611:2010.

Skin cooling unit 20 may contain an inorganic substance. Specifically, skin cooling unit 20 preferably includes at least one selected from the group consisting of Al₂O₃, ZnO, ZrO₂, MgO, GaN, AlN, and diamond. Since these materials have a high refractive index and a high thermal conductivity, the translucency and thermal conductivity of skin cooling unit 20 can be improved. Note that, Al₂O₃ (sapphire) has a refractive index of 1.79 and a thermal conductivity of 42 W/mK. ZnO has a refractive index of 2.01 and a thermal conductivity of 20 W/mK. ZrO₂ has a refractive index of 2.13 and a thermal conductivity of 3 W/mK. MgO has a refractive index of 1.74 and a thermal conductivity of 47 W/mK. GaN has a refractive index of 2.346 and a thermal conductivity of 200 W/mK. AlN has a refractive index of 2.175 and a thermal conductivity of 150 W/mK. Diamond has a refractive index of 2.417 and a thermal conductivity of 1000 W/mK.

Skin cooling unit 20 may contain a resin such as a silicone resin from a viewpoint of heat resistance and translucency. Further, skin cooling unit 20 may include a resin such as a silicone resin and a highly thermally conductive filler dispersed in the resin. Since skin cooling unit 20 includes the highly thermally conductive filler, heat of skin cooling unit 20 is easily dissipated, and thus skin S can be effectively cooled. The highly thermally conductive filler may contain an inorganic substance as described above.

A proportion of the inorganic substance in skin cooling unit 20 is preferably more than or equal to 10 mass%. By setting the proportion of the inorganic substance in skin cooling unit 20 to more than or equal to 10 mass%, the thermal conductivity of skin cooling unit 20 can be improved. The proportion of the inorganic substance in skin cooling unit 20 is more preferably more than or equal to 30 mass%, still more preferably more than or equal to 50 mass%, particularly preferably more than or equal to 70 mass%, and most preferably more than or equal to 90 mass%.

Skin cooling unit 20 is preferably cooled to be from -5°C to 35°C inclusive. By cooling skin cooling unit 20 to be more than or equal to -5°C, it is possible to cool skin S so that pain in skin S caused by the cooling is unlikely to occur. On the other hand, when skin cooling unit 20 is cooled to be less than or equal to 35°C, inflammation due to a rise in skin temperature during light irradiation can be suppressed. Skin cooling unit 20 is more preferably cooled to be more than or equal to 0°C, still more preferably more than or equal to 5°C, and particularly preferably more than or equal to 10°C. Further, skin cooling unit 20 is more preferably cooled to be less than or equal to 30°C, still more preferably less than or equal to 25°C, particularly preferably less than or equal to 20°C, and most preferably less than or equal to 15°C.

Temperature sensor 13 detects a temperature of skin S of the user. Temperature sensor 13 is provided so as to face skin cooling unit 20. Specifically, temperature sensor 13 is provided on substrate 12. In the present exemplary embodiment, temperature sensor 13 includes a non-contact temperature sensor such as a radiation thermometer.

Push switch 30 is a self-reset switch. Push switch 30 is provided at connection unit 42 of cooler 40. Push switch 30 is disposed outside light source 10 and skin cooling unit 20 in front-back direction X and width direction Y, and is provided so as to surround light source 10 and skin cooling unit 20. Push switch 30 includes two bases 31 and pressing unit 32.

Two bases 31 are fixed to connection unit 42 outside grip unit 43 of cooler 40 such that light source 10 and skin cooling unit 20 are disposed therebetween in width direction Y. Bases 31 have a quadrangular prism shape extending upward from connection unit 42.

Pressing unit 32 is engaged with bases 31, and moves in up-down direction Z by being pressed by skin S. Pressing unit 32 surrounds a periphery of light source 10 and skin cooling unit 20. Pressing unit 32 includes two first components 321 and one second component 322.

First component 321 has a columnar shape extending upward in up-down direction Z from bases 31, and is provided at a substantially central portion of bases 31 in front-back direction X and width direction Y, respectively. Second component 322 is disposed so as to be in contact with a surface of first component 321 opposite to bases 31. Second component 322 has a through hole at a center in front-back direction X and width direction Y, and has a donut shape extending in up-down direction Z. Light source 10 and skin cooling unit 20 are disposed in the through-hole of second component 322. A part of the surface of second component 322 protrudes upward in up-down direction Z from a surface of skin cooling unit 20 opposite to light source 10. Note that, in the present exemplary embodiment, first component 321 and second component 322 are different components separated from each other, but pressing unit 32 may be formed by one component that is continuously integrally formed. Further, the number of bases 31, first component 321, and second component 322 is not particularly limited, and can be changed as appropriate.

In a case where pressing unit 32 is not pressed, it protrudes toward a direction opposite to light source 10 (upward in up-down direction Z) against skin cooling unit 20 from a surface of skin cooling unit 20 in contact with skin S. Contact points (not illustrated) are provided inside bases 31 and pressing unit 32, and push switch 30 is configured such that while pressing unit 32 is not pressed, a circuit to which light source 10 is connected is opened without contact point between the contact point of bases 31 and the contact point of pressing unit 32. On the other hand, in a case where pressing unit 32 is pressed, a surface pressed by skin S moves toward the direction of light source 10 (downward in up-down direction Z) against skin cooling unit 20. Therefore, the contact point provided in bases 31 and the contact point provided in pressing unit 32 come into contact with each other, whereby the circuit to which light source 10 is connected is closed.

An elastic body (not illustrated) is provided between bases 31 and pressing unit 32. In a case where pressing unit 32 is pressed, the elastic body is elastically deformed, and pushes back pressing unit 32 by an elastic force generated by an elastic deformation. Therefore, when the force that presses pressing unit 32 is removed, the elastic body acts on pressing unit 32 so as to return pressing unit 32 to its original position, and thus a contact surface of pressing unit 32 with skin S moves toward a direction opposite to bases 31 (upward in up-down direction Z).

Push switch 30 switches between emission and non-emission of light from light source 10 such that light is emitted from light source 10 during at least a part of time while pressing unit 32 is pressed, and light is not emitted from light source 10 while pressing unit 32 is not pressed. Therefore, it is configured such that skin S is irradiated with light during at least a part of time while light irradiation hair removal device 1 is pressed against skin S, and the irradiation of light is stopped when light irradiation hair removal device 1 is separated from skin S.

Light may be emitted immediately after push switch 30 is pressed, or may be emitted after a predetermined time has elapsed since push switch 30 was pressed. A timing at which light is emitted from light source 10 may be controlled by controller 50. Light irradiation hair removal device 1 may emit light from light source 10 after skin S comes into contact with a surface of skin cooling unit 20. As a result, the skin surface is irradiated with light in a cooled state. Therefore, since heat generation of skin S is suppressed, irritation to the skin S can be suppressed. Further, since skin S is irradiated with light in contact with skin cooling unit 20, an uneven irradiation can be suppressed, and a stable hair removal effect can be obtained.

Cooler 40 cools skin cooling unit 20. Since light irradiation hair removal device 1 includes cooler 40, the temperature of skin cooling unit 20 can be further lowered. Therefore, a skin cooling effect by skin cooling unit 20 can be further improved. Cooler 40 includes heat dissipation unit 41 and air blower 45.

Heat dissipation unit 41 is connected to skin cooling unit 20, and dissipates heat taken from skin cooling unit 20. Heat dissipation unit 41 includes connection unit 42, grip unit 43, and heat dissipation fin 44.

Connection unit 42 is a plate-shaped member. Substrate 12 is provided on a first surface, which is one surface of connection unit 42. Substrate 12 is smaller than connection unit 42, and is provided so as to be accommodated inside connection unit 42. Grip unit 43 and push switch 30 are connected to outside of substrate 12 on the first surface of connection unit 42. Heat dissipation fin 44 is provided on a second surface, which is a surface opposite to the first surface of connection unit 42.

Grip unit 43 protrudes upward in up-down direction Z from the first surface of connection unit 42, and grips an entire peripheral edge of skin cooling unit 20. Therefore, light source 10 is surrounded by skin cooling unit 20, grip unit 43, and connection unit 42. The heat generated by light source 10 is dissipated via skin cooling unit 20 and heat dissipation unit 41 of cooler 40. Note that, while grip unit 43 grips the entire peripheral edge of skin cooling unit 20, grip unit 43 may be connected to at least a part of skin cooling unit 20.

Heat dissipation fin 44 is provided on the second surface of connection unit 42, which is a surface opposite to light source 10. Therefore, the heat of skin cooling unit 20 moves to heat dissipation fin 44 through grip unit 43 and connection unit 42. Heat dissipation fin 44 includes a plurality of fins, and has a large contact area with air, so that heat is easily dissipated.

Heat dissipation unit 41 preferably contains a material excellent in thermal conductivity. The value of the thermal conductivity of heat dissipation unit 41 may be larger than the value of the thermal conductivity of skin cooling unit 20. Specifically, heat dissipation unit 41 may contain a metal such as aluminum, iron, or copper. Grip unit 43, connection unit 42, and heat dissipation fin 44 may be made of the same material, or may be made of different materials.

Air blower 45 cools heat dissipation unit 41 by sending air to heat dissipation unit 41. Air blower 45 includes, for example, a fan, and when the fan rotates, an air flow is generated. Housing 5 is provided with a plurality of first opening portions 6 at positions facing air blower 45. Further, housing 5 is provided with a plurality of second opening portions 7 at positions facing heat dissipation fin 44. Therefore, when air blower 45 is driven, air taken in from outside of housing 5 through the plurality of first opening portions 6 is sent to heat dissipation fin 44. The heat of the air in contact with heat dissipation fin 44 is exchanged with the heat of heat dissipation fin 44, and heat dissipation fin 44 is cooled. The air heated in contact with heat dissipation fin 44 is discharged to outside of housing 5 through the plurality of second opening portions 7.

Note that, in the present exemplary embodiment, it has been described that light irradiation hair removal device 1 cools skin cooling unit 20 using air-cooled cooler 40, but skin cooling unit 20 may be cooled using a Peltier element or the like in addition to air-cooled cooler 40 or instead of air-cooled cooler 40. Light irradiation hair removal device 1 in a case of cooling skin cooling unit 20 using the Peltier element can cool skin cooling unit 20 more strongly.

Fig. 5 is a control block diagram according to controller 50. Controller 50 controls emission and non-emission of light from light source 10. Further, controller 50 controls a drive and stop of air blower 45. As shown in Fig. 5, temperature sensor 13 and push switch 30 are connected to an input side of controller 50. On the other hand, light source 10 and cooler 40 are connected to an output side of controller 50. Controller 50 has a computer system including a central processing unit (CPU), a read only memory (ROM), and a random access memory (RAM). Then, when the CPU executes programs stored in the ROM, the computer system functions as controller 50. Here, the programs executed by the CPU are recorded in advance in the ROM of the computer system, but may be provided by being recorded in a non-transitory recording medium such as a memory card, or may be provided through a telecommunication line such as the Internet.

Controller 50 turns on or blinks light source 10 in a case where push switch 30 is pressed. Controller 50 may cause light source 10 to emit light simultaneously when push switch 30 is pressed, or may cause light source 10 to emit light after a predetermined time period has elapsed since push switch 30 was pressed.

Controller 50 may receive signals related to the temperature of skin S from temperature sensor 13, and control the irradiation time of first irradiation light L1 by light source 10 according to the signals. As a result, since the irradiation of first irradiation light L1 can be stopped before the temperature of skin S becomes excessive, skin irritation can be reduced. Preferably, controller 50 controls the irradiation time of first irradiation light L1 such that the temperature of skin S is from -5°C to 35°C inclusive. By controlling the temperature of skin S to be more than or equal to -5°C, the pain in skin S associated with cooling can be less likely to occur. On the other hand, by controlling the temperature of skin S to be less than or equal to 35°C, it is possible to reduce skin irritation associated with light irradiation.

### [Operation]

The following describes operations and actions of light irradiation hair removal device 1 configured as described above.

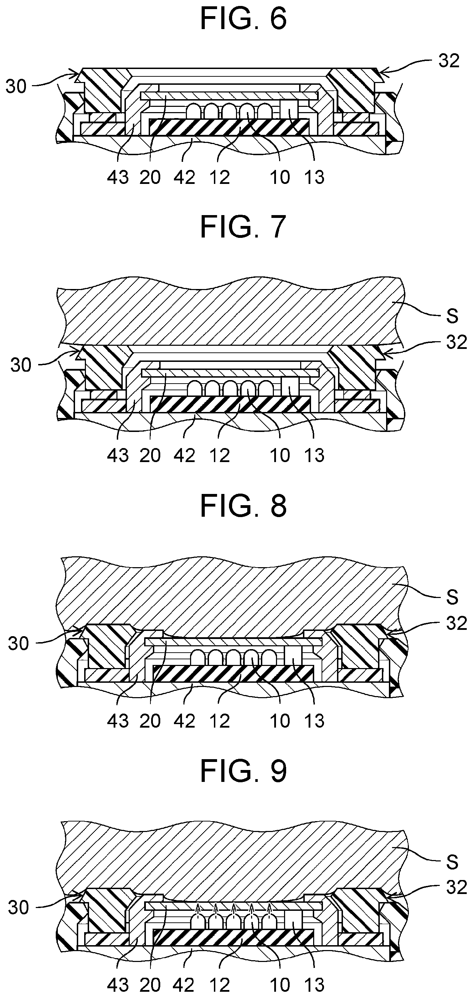

With reference to Figs. 6 to 9, a state in which light is emitted by light irradiation hair removal device 1 will be described. Fig. 6 is a cross-sectional view illustrating one example of a state before use of light irradiation hair removal device 1. Fig. 7 is a cross-sectional view illustrating one example of a state of light irradiation hair removal device 1 before push switch 30 is pressed. Fig. 8 is a cross-sectional view illustrating one example of a state of light irradiation hair removal device 1 after push switch 30 is pressed. Fig. 9 is a cross-sectional view illustrating one example of a state of light irradiation hair removal device 1 while skin S is irradiated with light.

As shown in Fig. 6, push switch 30 is not pressed before use of light irradiation hair removal device 1. Therefore, pressing unit 32 of push switch 30 protrudes toward a direction opposite to light source 10 against skin cooling unit 20 from a surface of skin cooling unit 20 in contact with skin S. In this state, light is not emitted from light source 10.

As shown in Fig. 7, when light irradiation hair removal device 1 is used, skin S of the user is pressed against light irradiation hair removal device 1. Pressing unit 32 of push switch 30 protrudes from a skin contact surface of skin cooling unit 20. Therefore, skin S of the user first comes into contact with push switch 30, and light source 10 is surrounded by skin S and push switch 30.

As shown in Fig. 8, push switch 30 is pressed in a state of being in contact with skin S. Specifically, in a case where pressing unit 32 of push switch 30 is pressed, the surface pressed by skin S moves toward the direction of light source 10 against skin cooling unit 20. As a result, skin S comes into contact with skin cooling unit 20 in a state where light source 10 is surrounded by skin S and push switch 30, and skin cooling unit 20 is shielded by skin S. Then, skin S is cooled by coming into contact with skin cooling unit 20.

As shown in Fig. 9, a circuit to which light source 10 is connected is closed by push switch 30, and light is emitted from light source 10. Since skin cooling unit 20 is shielded by skin S, and light source 10 is also surrounded by push switch 30, skin S is irradiated with light emitted from light source 10 without leakage. In order to bring skin cooling unit 20 and skin S into contact with each other more reliably, light may be emitted from light source 10 after a predetermined time period has elapsed since pressing unit 32 of push switch 30 was pressed.

### [Effect]

As described above, light irradiation hair removal device 1 according to the present exemplary embodiment includes light source 10, skin cooling unit 20, and push switch 30. Light source 10 intermittently emits light including first irradiation light L1 and second irradiation light L2 emitted for a longer time than first irradiation light L1 before first irradiation light L1, and having a wavelength from 400 nm to 1200 nm inclusive. Skin cooling unit 20 faces light source 10, transmits light emitted from light source 10, and cools skin S in a case where it comes into contact with skin S. Push switch 30 includes pressing unit 32 surrounding a periphery of light source 10 and skin cooling unit 20. In a case where pressing unit 32 is not pressed, it protrudes toward a direction opposite to light source 10 against skin cooling unit 20 from a surface of skin cooling unit 20 in contact with skin S. In a case where pressing unit 32 is pressed, the surface pressed by skin S moves toward the direction of light source 10 against skin cooling unit 20. Push switch 30 switches between emission and non-emission of light from light source 10 such that light is emitted from light source 10 during at least a part of time while pressing unit 32 is pressed, and light is not emitted from light source 10 while pressing unit 32 is not pressed. First irradiation light L1 has a maximum irradiance that is the largest irradiance of light emitted by light source 10 in an intermittent manner, and second irradiation light L2 has an irradiance smaller than the maximum irradiance.

As a result, light irradiation hair removal device 1 gently performs preheating with less skin irritation by second irradiation light L2, and applies heat having a good hair removal promoting effect by first irradiation light L1. As a result, light irradiation hair removal device 1 can reduce skin irritation regardless of individual properties of the user as compared with a case where light having the same irradiance is emitted alone while maintaining the hair removal effect. Therefore, light irradiation hair removal device 1 can reduce discomfort associated with light irradiation.

Further, light irradiation hair removal device 1 can irradiate skin S with light in a state where light source 10 is surrounded by push switch 30 and skin S. Therefore, light irradiation hair removal device 1 can suppress leakage of light. Further, in light irradiation hair removal device 1, skin cooling unit 20 can come into contact with skin S to cool skin S at the time of light irradiation. Therefore, light irradiation hair removal device 1 can suppress inflammation of skin S.

Note that, light irradiation hair removal device 1 may include a near-infrared LED (light source 10), a push-in irradiation switch (push switch 30), and a skin cooling unit (skin cooling unit 20). The skin cooling unit (skin cooling unit 20) is made of a transparent material that is cooled on a light beam that is an upper portion of the near-infrared LED (light source 10) and transmits light of the near-infrared LED (light source 10). Light irradiation hair removal device 1 has a configuration in which the near-infrared LEDs (light source 10) emit light after a top surface of the skin cooling unit (skin cooling unit 20) comes into contact with the skin (skin S) by being pushed into the skin (skin S). In addition, light irradiation hair removal device 1 is characterized in that light source 10 is controlled so that light quantity of the near-infrared LED (light source 10) becomes high from low. Even with such light irradiation hair removal device 1, it is possible to reduce discomfort associated with light irradiation.

As in light irradiation hair removal device 1 according to the present exemplary embodiment, the irradiance of light emitted from light source 10 may be more than 0 W/cm² and less than or equal to 50 W/cm².

As a result, light irradiation hair removal device 1 can suppress a rise in skin temperature due to light irradiation, and can reduce skin irritation.

As in light irradiation hair removal device 1 according to the present exemplary embodiment, the irradiation time of each light emitted from light source 10 in an intermittent manner, which is the total irradiation time of each light in one cycle, may be from 500 ms to 1000 ms inclusive.

As a result, light irradiation hair removal device 1 can achieve a good hair removal effect on hair from an early growth period to a growth period while suppressing a rise in the skin temperature, and can reduce the skin irritation.

As in light irradiation hair removal device 1 according to the present exemplary embodiment, the irradiation time of first irradiation light L1 may be more than 0 ms and less than 500 ms, and the irradiation time of second irradiation light L2 may be longer than the irradiation time of first irradiation light L1 of more than 0 ms and less than or equal to 500 ms.

As a result, light irradiation hair removal device 1 can increase the temperature of hair follicles while reducing the irritation to skin S due to light irradiation. Therefore, light irradiation hair removal device 1 can promote hair removal while more reliably reducing discomfort associated with light irradiation.

### (Other exemplary embodiments)

As described above, the above exemplary embodiment has been described as an example of the technology in the present disclosure. However, the technology of the present disclosure is not limited to them, and is also applicable to exemplary embodiments in which changes, replacements, additions, omissions, and the like are made. Thus, hereinafter, other exemplary embodiments are illustrated as examples.

Note that, light irradiation hair removal device 1 according to the above exemplary embodiment has been described as an example that light source 10 includes LEDs. However, it is sufficient that light source 10 can emit light having a wavelength from 400 nm to 1200 nm inclusive. Light source 10 is not limited to LEDs, and may include, for example, a xenon lamp, laser diodes, and a combination thereof. However, if LEDs are used as light source 10, light irradiation hair removal device 1 can be miniaturized as described above.

Further, light irradiation hair removal device 1 according to the above exemplary embodiment has been described as an example that it cools skin cooling unit 20 using cooler 40. However, in a case where the thermal conductivity of skin cooling unit 20 is high, it is not always necessary to cool skin cooling unit 20 using cooler 40, since the heat dissipation of skin cooling unit 20 is high.

Further, light irradiation hair removal device 1 according to the above exemplary embodiment has been described as an example that cooler 40 is connected to skin cooling unit 20 to cool skin cooling unit 20. However, cooler 40 does not need to be connected to skin cooling unit 20.

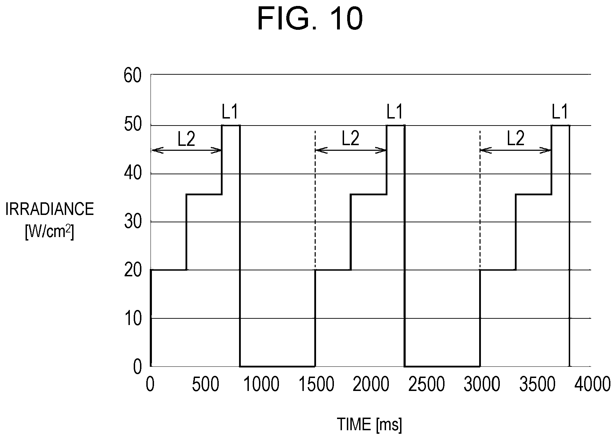

Here, a modification of the irradiation illuminance of second irradiation light L2 will be described with reference to Fig. 10. Fig. 10 is a graph illustrating another example of the relationship between the irradiation time and the irradiance of light emitted from light source 10.

In the above exemplary embodiment, an example in which second irradiation light L2 has one irradiance of 20 W/cm² has been described. However, as shown in Fig. 10, second irradiation light L2 may have two or more different irradiance. Specifically, to start with, second irradiation light L2 is emitted from light source 10. Second irradiation light L2 includes light having an irradiance of 20 W/cm² and light having an irradiance of 35 W/cm². After light having an irradiance of 20 W/cm² is emitted for 300 ms, light having an irradiance of 35 W/cm² is emitted for 300 ms. Next, first irradiation light L1 is emitted from light source 10 following the emission of second irradiation light L2. The irradiance of first irradiation light L1 is 50 W/cm², and the irradiation time of first irradiation light L1 is 200 ms. Therefore, total irradiation time of second irradiation light L2 and first irradiation light L1 is 800 ms. After second irradiation light L2 and first irradiation light L1 are emitted, light emission by light source 10 is stopped. Off time of light source 10 is 700 ms. Next, second irradiation light L2 and first irradiation light L1 are emitted again from light source 10 in this order. Light source 10 intermittently emits light including second irradiation light L2 and first irradiation light L1 by repeatedly turning on and off light including second irradiation light L2 and first irradiation light L1. Even in a case where light is emitted in this manner, light irradiation hair removal device 1 according to this modification can reduce discomfort associated with light irradiation.

Note that, in Fig. 10, an example in which the irradiance of second irradiation light L2 increases with the lapse of time has been described. However, second irradiation light L2 only needs to have an irradiance smaller than that of first irradiation light L1, and second irradiation light L2 may include a plurality of light, the irradiance of which decreases with the lapse of time. However, as shown in Fig. 10, in a case where light source 10 emits light in an intermittent manner so that the irradiance increases with the lapse of time, the user is easily used to light irradiation, and thus light irradiation hair removal device 1 according to this modification can further reduce discomfort associated with light irradiation.

Further, in Figs. 4 and 10, light of the same pattern is repeatedly emitted in an intermittent manner, but the pattern of each light emitted in an intermittent manner may not necessarily be the same, and light of different patterns may be emitted in an intermittent manner.

Note that, since the above-described exemplary embodiments are intended to illustrate the technique in the present disclosure, various changes, replacements, additions, omissions, and the like can be made within the scope of claims.

### INDUSTRIAL APPLICABILITY

As described above, the light irradiation hair removal device according to the present disclosure can be applied to, for example, a light irradiation hair removal device for business use and home use.

### REFERENCE MARKS IN THE DRAWINGS

1: light irradiation hair removal device
5: housing
6: first opening portion
7: second opening portion
10: light source
12: substrate
13: temperature sensor
20: skin cooling unit
30: push switch
31: base
32: pressing unit
40: cooler
41: heat dissipation unit
42: connection unit
43: grip unit
44: heat dissipation fin
45: air blower
50: controller
321: first component
322: second component
L1: first irradiation light
L2: second irradiation light
S: skin

## Claims

1. A light irradiation hair removal device comprising:
a light source (10) configured to intermittently emit light including a first irradiation light and a second irradiation light, the second irradiation light being emitted for a longer time than the first irradiation light and before the first irradiation light, the first irradiation light and the second irradiation light each having a wavelength from 400 nm to 1200 nm inclusive;
a skin cooling unit (20) that faces the light source (10) and is configured to transmit light emitted from the light source, and to cool
a skin in a case where it comes into contact with the skin; and
a push switch (30) that includes a pressing unit (32) surrounding a periphery of the light source and the skin cooling unit; wherein
when the pressing unit is not being pressed, the pressing unit protrudes toward a direction opposite to the light source against the skin cooling unit from a surface of the skin cooling unit, the surface that is to be brought into contact with the skin,
when the pressing unit is pressed by the skin, a surface of which pressed by the skin moves toward a direction of the light source against the skin cooling unit, and
the push switch is configured to switch between emission and non-emission of light from the light source such that light is emitted from the light source during at least a part of time while the pressing unit is pressed, and light is not emitted from the light source while the pressing unit is not pressed,
wherein
the first irradiation light has a maximum irradiance that is a largest irradiance of the light emitted by the light source in an intermittent manner, and the second irradiation light has an irradiance smaller than the maximum irradiance.

2. The light irradiation hair removal device according to Claim 1, wherein an irradiance of the light emitted from the light source is more than 0 W/cm² and less than or equal to 50 W/cm².

3. The light irradiation hair removal device according to Claim 1 or 2, wherein total irradiation time in one cycle of the light emitted from the light source in an intermittent manner is from 500 ms to 1000 ms inclusive.

4. The light irradiation hair removal device according to any one of Claims 1 to 3, wherein an irradiation time of the first irradiation light is more than 0 ms and less than 500 ms, and an irradiation time of the second irradiation light is longer than the irradiation time of the first irradiation light of more than 0 ms and less than or equal to 500 ms.

## Patentansprüche

1. Vorrichtung für Entfernung von Haaren mittels Lichtbestrahlung, die umfasst:
eine Lichtquelle (10), die so ausgeführt ist, dass sie intermittierend Licht emittiert, das ein erstes Bestrahlungslicht und ein zweites Bestrahlungslicht einschließt, wobei das zweite Bestrahlungslicht über eine längere Zeit als das erste Bestrahlungslicht und vor dem ersten Bestrahlungslicht emittiert wird, und das erste Bestrahlungslicht sowie das zweite Bestrahlungslicht jeweils eine Wellenlänge von 400 nm bis einschließlich 1.200 nm haben;
eine Einheit (20) zum Kühlen der Haut, die der Lichtquelle (10) zugewandt und so ausgeführt ist, dass sie von der Lichtquelle emittiertes Licht durchlässt und eine Haut dort kühlt, wo sie in Kontakt mit der Haut kommt; sowie
einen Druckschalter (30), der eine Press-Einheit (32) enthält, die einen Umfang der Lichtquelle und der Einheit zum Kühlen der Haut umschließt; wobei
wenn die Press-Einheit nicht gepresst wird, die Press-Einheit in einer Richtung entgegengesetzt zu der Lichtquelle zu der Einheit zum Kühlen der Haut von einer Fläche der Einheit zum Kühlen der Haut, d.h. der Fläche, die in Kontakt mit der Haut zu bringen ist, vorsteht,
wenn die Press-Einheit durch die Haut gepresst wird, eine Fläche derselben, die durch die Haut gepresst wird, sich in einer Richtung der Lichtquelle zu der Einheit zum Kühlen der Haut bewegt, und
der Druckschalter so ausgeführt ist, dass er zwischen Emission und Nicht-Emission von Licht von der Lichtquelle so umschaltet, dass Licht von der Lichtquelle während wenigstens eines Teils der Zeit emittiert wird, während der die Press-Einheit gepresst wird, und kein Licht von der Lichtquelle emittiert wird, während die Press-Einheit nicht gepresst wird, und
wobei
das erste Bestrahlungslicht eine maximale Bestrahlungsstärke hat, die eine größte Bestrahlungsstärke des von der Lichtquelle intermittierend emittierten Lichtes ist, und das zweite Bestrahlungslicht eine Bestrahlungsstärke hat, die kleiner ist als die maximale Bestrahlungsstärke.

2. Vorrichtung für Entfernung von Haaren mittels Lichtbestrahlung nach Anspruch 1, wobei eine Bestrahlungsstärke des von der Lichtquelle emittierten Lichtes mehr als 0 W/cm² und 50 W/cm² oder weniger beträgt.

3. Vorrichtung für Entfernung von Haaren mittels Lichtbestrahlung nach Anspruch 1 oder 2, wobei die Gesamt-Bestrahlungszeit in einem Zyklus des von der Lichtquelle intermittierend emittierten Lichtes zwischen 500 ms bis einschließlich 1.000 ms beträgt.

4. Vorrichtung für für Entfernung von Haaren mittels Lichtbestrahlung nach einem der Ansprüche 1 bis 3, wobei eine Bestrahlungszeit des ersten Bestrahlungslichtes mehr als 0 ms und weniger als 500 ms beträgt und eine Bestrahlungszeit des zweiten Bestrahlungslichtes länger ist als die Bestrahlungszeit des ersten Bestrahlungslichtes von mehr als 0 ms und 500 ms oder weniger.

## Revendications

1. Dispositif d'épilation par irradiation lumineuse comprenant :
une source de lumière (10) configurée pour émettre de la lumière par intermittence incluant une première lumière d'irradiation et une seconde lumière d'irradiation, la seconde lumière d'irradiation étant émise sur une plus longue durée que la première lumière d'irradiation et avant la première lumière d'irradiation, la première lumière d'irradiation et la deuxième lumière d'irradiation ayant chacune une longueur d'onde entre 400 nm et 1 200 nm inclus ;
une unité de refroidissement de la peau (20) qui fait face à la source de lumière et qui est configurée pour transmettre la lumière émise par la source de lumière, et pour refroidir une peau dans un cas où elle entre en contact avec la peau ; et
un interrupteur à poussoir (30) qui inclut une unité de pression (32) entourant une périphérie de la source de lumière et de l'unité de refroidissement de la peau ; dans lequel
lorsque l'unité de pression n'est pas pressée, l'unité de pression fait saillie vers une direction opposée à la source de lumière contre l'unité de refroidissement de la peau à partir d'une surface de l'unité de refroidissement de la peau, la surface qui doit être portée en contact avec la peau,
lorsque l'unité de pression est pressée par la peau, dont une surface pressée par la peau se déplace vers une direction de la source de lumière contre l'unité de refroidissement de la peau, et
l'interrupteur à poussoir est configuré pour commuter entre l'émission et la non-émission de lumière par la source de lumière de sorte que de la lumière est émise depuis la source de lumière pendant au moins une partie du temps lorsque l'unité de pression est pressée, et que de la lumière n'est pas émise par la source de lumière lorsque l'unité de pression n'est pas pressée ; dans lequel
la première lumière d'irradiation présente un éclairement énergétique maximal qui est un éclairement énergétique le plus grand de la lumière émise par la source de lumière par intermittence, et la seconde lumière d'irradiation présente un éclairement énergétique inférieur à l'éclairement énergétique maximal.

2. Dispositif d'épilation par irradiation lumineuse selon la revendication 1, dans lequel un éclairement énergétique de la lumière émise depuis la source de lumière est supérieur à 0 W/cm² et inférieure ou égal à 50 W/cm².

3. Dispositif d'épilation par irradiation lumineuse selon la revendication 1 ou 2, dans lequel une durée d'irradiation totale en un cycle de la lumière émise depuis la source de lumière par intermittence est de 500 ms à 1000 ms inclus.

4. Dispositif d'épilation par irradiation lumineuse selon l'une quelconque des revendications 1 à 3, dans lequel une durée d'irradiation de la première lumière d'irradiation est supérieure à 0 ms et inférieure à 500 ms, et une durée d'irradiation de la seconde lumière d'irradiation est plus longue que la durée d'irradiation de la première lumière d'irradiation de plus de 0 ms et inférieure ou égale à 500 ms.
